# EUROPEAN PATENT SPECIFICATION

(11) **EP 2 010 082 B1**
(45) Date of publication and mention of the grant of the patent: **17.11.2010**
(21) Application number: 07760364.5
(22) Date of filing: 10.04.2007
(51) Int. Cl.: A61B 17/70

(54) **CONNECTOR APPARATUS**
VERBINDERVORRICHTUNG
APPAREIL CONNECTEUR

(30) Priority: 24.04.2006 US 409823
(43) Date of publication of application: 07.01.2009
(73) Proprietor: Warsaw Orthopedic, Inc., Warsaw, IN 46581 (US)
(72) Inventor: REZACH, William A., Atoka Trail, Tennessee 38004 (US)
(74) Representative: Hutchinson, Glenn Stanley
(86) International application number: PCT/US2007/066286
(87) International publication number: WO 2007/127602

(56) References cited:
- DE-A1- 4 434 574
- FR-A- 2 763 830
- US-A- 5 630 816
- US-A- 6 136 002
- US-A1- 2005 228 378
- US-A1- 2006 079 892

## Description

The present disclosure broadly concerns spinal fixation systems and generally relates to a connector apparatus used to connect spinal rods. The apparatus can be useful for correction of spinal injuries or deformities.

### BACKGROUND

Several techniques and systems have been developed for use in correcting and stabilizing spinal curvatures, and for facilitating spinal fusion in the case of spinal disorders or degenerative conditions. In some systems, a pair of bendable rods may be longitudinally disposed adjacent the column and are fixed to various vertebrae along the length of the spine by way of a number of fixation elements, such as hooks and screws, in certain situations, it is desirable to supplement an existing spinal rod connected to the vertebral column with a new spinal rod, to add strength and stability to the fixation system.

Numerous spinal rod systems have been developed which provide transverse connectors for linking the adjacent spinal rods across the spinal midline to provide a rigid and stable construct. Such systems can present one or more difficulties for spinal surgeons. Many of the devices are high profile n which increases soft tissue trauma and surgical complications. Moreover, in certain situations it is desirable to provide a transverse connection between adjacent spinal rods on the same side of the spinal midline.

Rigid transverse connections between spinal rods are beneficial because they restrict rod migration and increase construct stiffness. In many cases involving multi-level fusion of the spine, these features are essential while solid bone fusion is accomplished In the post-operative period before fusion occurs, a significant amount of motion can occur between rods or other elongated members and other structure such as wires and hooks. That motion can, for example, allow a scoliotic correction to decrease or the pelvis to de-rotate towards a previous, deformed position. By providing a rigid transverse connection between two spinal rods, the loss of correction can be reduced and a stiffer construct can be created which may enhance the promotion of a solid fusion.

US2006/0079892 A1 discloses a spinal connector for interconnecting rods according to the preamble of claim 1, in which two mating members can pivot and/or translate within the coronal plane of the subject to adjust to the positions of the rods.

Examples of other known spinal rod connectors are disclosed in US2005/0228378, US5630816 and FR2763830.

A need remains for low profile devices which link adjacent spinal rods in an easy-loading, top-lightening fashion with a minimum of components and steps, providing increased stability to the fixation system.

### SUMMARY OF THE INVENTION

The present invention solves these problems and provides a connector apparatus as defined by independent claim 1. Preferred embodiments are defined by the appended dependent claims.

### BRIEF DESCRIPTION OF THE DRAWINGS

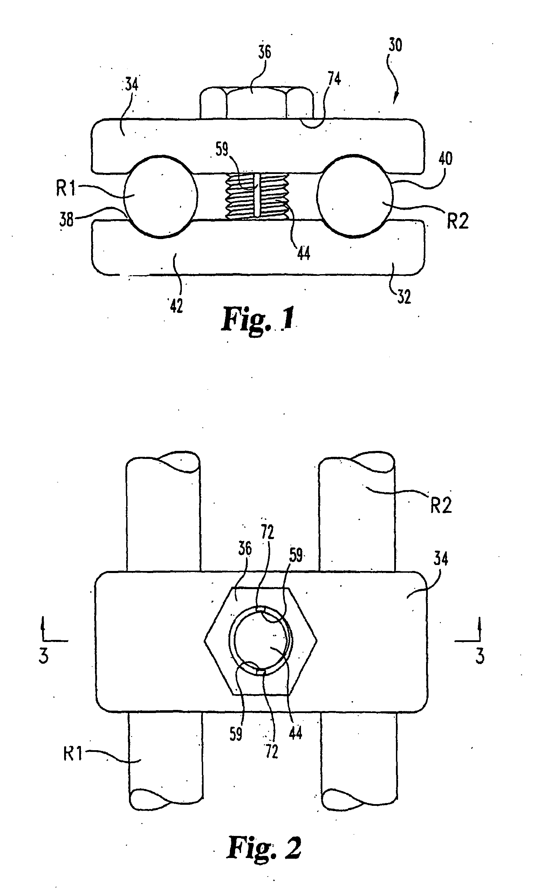

FIG. 1 is a side elevational view of an embodiment of a connector device connected to elongated members.
FIG. 2 is a top plan view of the embodiment shown in FIG. 1.

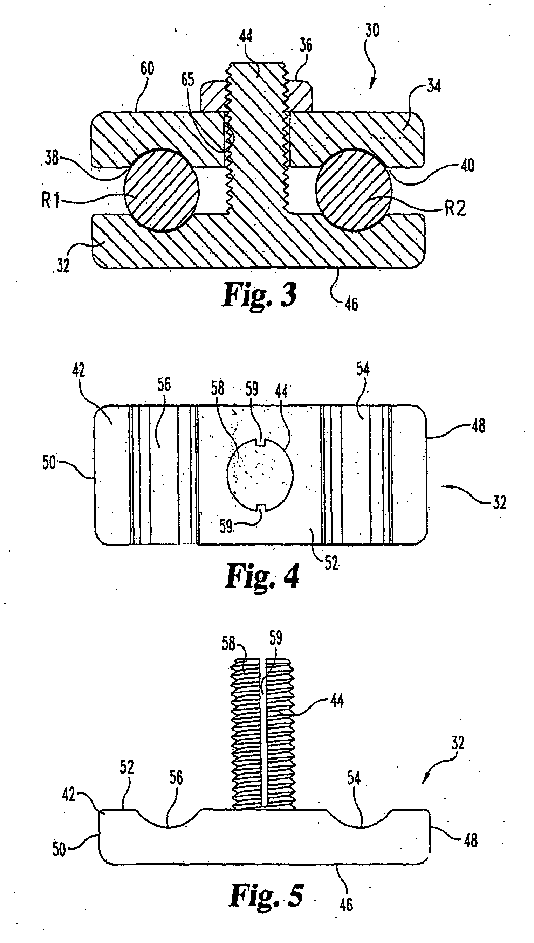

FIG. 3 is a cross-sectional view of the embodiment of FIG. 1, taken along the lines 3---3 in FIG. 2 and viewed in the direction of the arrows.
FIG. 4 is a top plan view of an embodiment of a first member of the embodiment shown in FIG. 1
FIG. 5. is a side elevational view of the embodiment shown in FIG 4.
FIG. 6 is a side elevational view of an embodiment of a second member of the embodiment shown in FIG. 1.
FIG. 7 is a bottom plan view of the embodiment shown in FIG 6.
FIG. 8 is a side elevational view of an embodiment of a locking member that may be used with the embodiments of a connector device.
FIG. 9 is a bottom plan view of another embodiment of a second member of an embodiment of a connector device.
FIG. 10 is a top plan view of another embodiment of a first member of an embodiment of a connector device.

### DESCRIPTION OF THE ILLUSTRATED EMBODIMENTS

For the purpose of promoting an understanding of the principles of the disclosure, reference will now be made to the embodiments illustrated in the drawings and specific language will be used to describe the same it will nevertheless be understood that no limitation of the scope of the claims is thereby intended, such alterations and further modifications in the illustrated device, and such further applications of the principles of the disclosure as illustrated therein, being contemplated as would normally occur to one skilled in the art to which the disclosure relates.

Referring generally to the figures, there is shown an embodiment of a connector apparatus 30. In that embodiments, apparatus 30 includes a first holding member 32, a second holding member 34, and a locking member 36. Generally, members 32 and 34 are assembled together to form passages 38 and 40 for rods R1 and R2, as further discussed below. Member 32 and 34 are secured together via locking member 36 in this embodiment.

Member 32 is generally T-shaped in the illustrated embodiment, having a base portion 42 and a post 44 extending from base 42. Post 44 may be formed integrally with each other, or in other embodiments may be separately fashioned and attached together, as by welding or other suitable attachment method. Post 44 is approximately centered with respect to portion 42 in the illustrated embodiment. In other embodiments, post 44 may be otherwise positioned, as for example placed toward one side of portion 42.

Base 42 is somewhat plate-like, having an external surface 46 and sides 48 and 50, and internal surface 52. At least part of surface 52 is concave in the illustrated embodiment, and in a particular embodiment surface 52 includes grooves or channels 54 and 56. As channels 54 and 56 can be substantially cylindrical and have a diameter of a size sufficient to accommodate rods R1, R2 or other elongated members of various sizes.

The rods R1, R2 have a diameter larger than the diameter of the channels 54,56 such that, in use, the rods contact the edges of the channels. Channels 54 and 56 are substantially parallel to each other in this embodiment, and are on either side of post 44. Channels 54 and 56 may be sized and configured essentially the same as each other, as shown in the illustrated embodiment, or one channel may be different from the other in terms of diameter, depth, or other dimension. Further, channels 54 and 56 could both be placed on one slide of post 44, and/or may be non-parallel.

Post 44, as noted above, extends from base 42, and in the illustrated embodiment has an axis that is substantially perpendicular to portion 42. Post 44 may be threaded with standard machine threads substantially along its existing length from its free end 58 to base 42. Post 44 has a diameter of a size that will allow placement of a rod in each of channels 54 and 56. Thus, in the illustrated embodiment, post 44 does not impinge on either of channels 54 or 56. in other embodiment, however, post 44 could enter one or both of channels 54 or 56. In such cases, a rod may contact post 44 as well as the surface and/or edge(s) of its respective channel. The post 44 includes at least one longitudinal groove 59. The illustrated embodiment of post 44 includes two grooves 59 that extend from free end 58 toward base 42, and in a particular embodiment extends substantially all the way along post 44. In other embodiments, post 44 may have a single groove 59, or more than two grooves 59. Where multiple grooves 59 are provided in post 44, they may be irregularly spaced around post 44.

Second holding member 34 may be thought of as a cover or cap that fits around post 44. The illustrated embodiment of member 34 is a plate-like structure having an exterior surface 60, and interior surface 62, sides 63 and 64, and a hole 65. Exterior surface 60 is substantially planar in the illustrated embodiment, but may have a curve in other embodiments, and may be substantially smooth or roughened. For example, exterior surface 60 could be knurled for a high-friction connection with locking member 36. interior surface 62 includes two channels 66 and 68, which can be substantially cylindrical and have a diameter of a size sufficient to accommodate rods or other elongated members of various sizes. Channels 66 and 68 are substantially parallel to each other and are on either side of hole 65. Channels 66 and 68 may be sized and configured essentially the same as each other, as shown in the illustrated embodiment, or one channel may be different from the other in terms of diameter, depth, or other dimension. Channels 66 and 68 could both be placed on one side of hole 65, and/or may be non-parallel. Further, one or both of channels 66 and 68 may be substantially identical to a respective facing channel 54 or 56. In the illustrated embodiment, for example, channel 66 is substantially identical to facing channel 54 at least in terms of diameter and depth, and channel 68 is substantially identical to facing channel 56 at least in terms of diameter and depth.

Hole 65 of member 34 may be substantially centered in member 34. Second holding member includes at least one boss 72. In the illustrated embodiment, member 34 has a substantially smooth inner surface 70, and a pair of bosses 72 extend radially into hole 65. Bosses 72 are positioned in hole 65 so that they act with grooves 59 as a keying mechanism, ensuring that member 34 can be placed around post 44 only in particular orientation (s). In a particular embodiment, bosses 72 and grooves 59 are positioned with regard to their respective members 34 and 32 so that member 32 can be assembled with member 34 only when members 32 and 34 are substantially aligned with each other. In embodiments in which members 32 and 34 include one or more channels, bosses 72 and grooves 59 may be positioned so that such channels in each member are aligned with each other. In embodiments in which post 44 includes a different number of grooves 59, a number of bosses 72 equal to or less than the number of grooves 59 may be provided in hole 65, or hole 65 and post 44 could be otherwise keyed together.

Lock member 36 is an internally-threaded nut in the illustrated embodiment with its internal threads being compatible with the machine threads on post 44. Lock member 36 may have an external hexagonal print for turning and tightening onto post 44. An underside 74 of lock member 36 may substantially conform to an adjacent portion of exterior surface 60 of member 34. Thus, where exterior surface 60 is substantially planar, underside 74 may also be substantially planar, and where exterior surface 60 has a curve adjacent hole 64 (e.g. a concave or conical surface), underside 74 may also have a substantially similar curve. Further, underside 74 may be smooth or roughened to provide close contact and/or high-friction contact with exterior surface 60. It will be seen that a variety or nut configurations could be used for lock member 36, such as a lock nut, or other types of a looking or holding member could be used, such as a press-on clamp or a shape-memory clamp.

Device 30 is generally used to connect two orthopedic elongated members, such as spinal rods, to each other. The use of device 30 is described below with respect to spinal surgical procedures. It will be understood that other uses could be made of device 30 in other orthopedic procedures.

In general, a surgeon first obtains access to the vertebrae or motion segment(s) to be instrumented via open, minimally-invasive or other techniques. A first rod R1 is connected to one or more vertebrae via bone anchors (not shown), which may be screws, hooks, clamps, other anchoring devices or a combination of them, and a second rod R2 is similarly connected to one or more of the same vertebrae as rod R1 or to a different set of vertebrae. Such attachment of rods via bone anchors to vertebrae can occur during the same surgery in which device 30 is implanted, or one or both of rods R1 and R2 can have been implanted in a surgery prior to introduction of device 30. In the latter case, it may be said that device 30 is placed in a "revision surgery," i.e. one that places new implant(s), removes or makes adjustments to old implant(s), makes adjustments or further corrections to the spinal column, or otherwise revises a prior surgical procedure.

Member 32 of device 30 is placed with respect to rods R1 and R2 so that rods R1 and R2 are adjacent to interior surface 52 and post 44 is between rods R1 and R2. In embodiments in which member 32 includes channels 54 and/or 56, one or both of rods R1 and R2 may be placed at least partially in or adjacent to respective such channels. In situations in which rod R1 is connected to vertebrae in a previous surgical procedure or is otherwise connected to vertebrae prior to placement of device 30, then member 32 may be maneuvered around rod R1 so that rod R1 is adjacent to member 32 as indicated above. For example, a side (e.g. side 50) of member 32 may be moved between a previously-fixed rod R1 and adjacent bone or other tissue. Rod R2, which may be a "revision" rod, is then placed with respect to member 32 as indicated above. Rod R2 may be attached to vertebrae prior to or after connection to device 30. In situations in which rods R1 and R2 are being implanted during the same surgical procedure, device 30 may be placed with respect to rods R1 and R2 prior to the rods' connection to vertebrae, after connection of one rod to vertebrae but before the connection of the other to vertebrae, or may be placed with respect to rods R1, R2 after their connection to vertebrae.

Member 34 is placed over member 32 so that post 44 of member 32 extends through hole 64 of member 34. Post 44 includes one or more grooves 59 and one or more bosses 72 extend into hole 65, Boss(es) 72 are aligned with groove(s) 59 so that boss(es) 72 slide along groove(s) as member 34 slides down along post 44. Member 34 is slid along post 44 so that it is adjacent or abutting rods R1 and R2, e.g. when rods R1 and R2 are at least partially in or adjacent to channels 66 and 68. When member 34 and member 32 are so positioned with respect to each other and rods R 1 and R2, lock member 36 can be engaged with post 44. In the embodiment in which lock member 36 is a nut and post 44 is threaded, lock member 36 is threaded onto post 44 and against member 34 in order to press members 32 and 34 against rods R1 and R2. If the height of post 44 is such that a portion of post 44 sticks out from lock member 36 when device 30 is locked to rods R1 and R2, then the portion of post 44 that stricks out can be cut or clipped off or otherwise removed.

As previously noted, with device 30 two rods R1 and R2 can be connected together to form either a sturdy dual-rod support for one set of vertebrae or vertebral motion segments, or to term a longer "rod" by linking together two rods, one of which is attached to a superior set of vertebrae and the other of which is attached to an inferior set of vertebrae. Device 30 is easy to position with respect to a rod previously implanted, since it can be positioned with respect to such a rod in available spaces, i.e. without moving bone anchors to which the rod is connected or removing or disturbing bone or other tissue that has grown around or into parts of the rod or anchors. If necessary, bone or other tissue can be placed between members 32 and 34, such as in channels 54, 56, 66, and/or 68, and members 32 and 34 can be locked around it and the rod members to which it is attached. The open nature of device 30 with the separation of members 32 and 34 and the easy access to the interior of device 30 allow use of device 30 in a range of anatomical or surgical situations.

It will be understood that variations to the above-described structures can be made. For example, embodiments of post 44 may be relatively smooth or include other features that permit connection of a locking mechanism. If post 44 is smooth, then a clamp mechanism (e.g. a spring-loaded clamp) could be placed around post 44 and atop member 34 so that members 32 and 34 are held around and/or to elongated members such as rods R1 and R2. apertures or indentations could be provided in post 44 into which a pin or bar can be inserted to hold a locking mechanism to post 44 and against member 34.

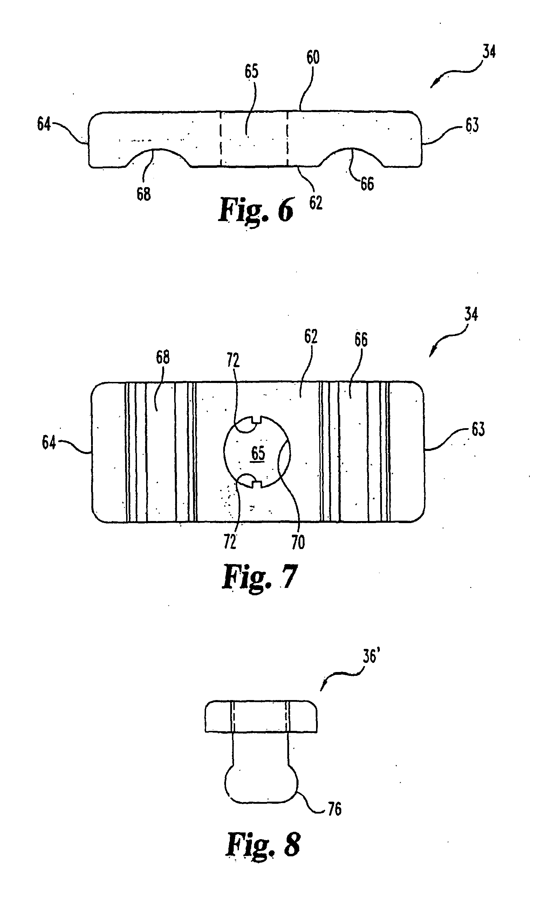

Further, where post 44 is threaded, an internally-threaded lock member 36', shown in one embodiment in FIG. 8, could be provided that includes an undersurface or extension 76. Lock member 36' not only exerts force on member 34 so that members 32 and 34 grip the elongated members, but also undersurface 76 contacts one or both elongated members. In this way, an additional point or points of engagement between the elongated members and connectors 20 are provided, and the friction and grip strength of the connector can be increased. Undersurface 76 may be a part of a compressible portion, for example a portion similar to a leaf spring or a portion of elastically compressible material such as a rubber or elastic polymer.

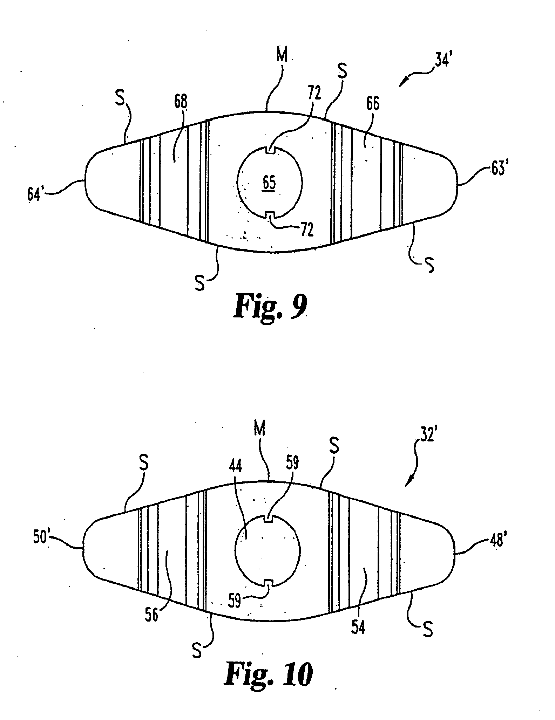

Other variations to the embodiments noted above are also possible. For example, members 32 and/or 34 may be substantially rectangular, or they may have other shapes. To make such members less bulky and less likely to interfere with or irritate adjacent tissue, members 32' and/or 34' may be substantially diamond-shaped, as seen in FIGS. 9-10. In that embodiment, members 32' and 34' are widest at a middle portion M proximate to hole 65 and post 44, and narrow to a relatively slim width at side portions 48', 50', 63' and 64' adjacent the elongated members. Such a diamond shape further provides surfaces S that are oblique to the longitudinal axis of an elongated member (shown in phantom). When flexion or extension of the vertebrae connected to the elongated member occurs, whether during surgery or in natural motion, stress on the elongated member is limited due to the non-perpendicular relationship between the elongated member and the adjacent surface S of member 32' and 34'. It will be seen that members 32' and 34' may have substantially the same shape, or one may be somewhat differently shaped from the other. Other aspects of members 32' and 34' may be substantially the same as aspect described above with respect to members 32 and 34.

Additionally, as noted above, where channels (e.g. channels 54, 56, 66 and 68) are provided, they may be sized substantially identically to each other, or they may be sized somewhat differently. Thus, for example, channel 54 in member 32 and channel 66 in member 34 may be somewhat smaller in radius than channel 56 in member 32 and channel 68 in member 34. In that situation, a larger rod could be accommodated in the passage created by channels 56 and 68, and a smaller rod could be accommodated in the passage created by channels 54 and 66. Such a configuration will be useful in situations in which implantation of rods of two different diameters is indicated. Thus, for example, where a relatively thick rod is implanted in a first surgery, and relatively minimal revision is necessary, a somewhat thinner rod may be implanted in a revision surgery with an embodiment of connector 30 designed as previously noted. Similarly, where a relatively thin rod is first implanted, perhaps in a pediatric case, a later thicker rod may be implanted in a revision with such an embodiment of connector 30.

The parts of connector 30 are composed of biocompatible materials that are also compatible with particular elongated members or other implants with which connector 30 will be used. Thus, connector 30 may be made of titanium, nickel, alloys of titanium and nickel, stainless steel, certain sturdy plastic materials, or other sturdy materials. The material(s) chosen for connector device 30 should be the same as those of the rods with which connector device 30 h used, or at least of a material that will not cause discomfort or an adverse reaction when used with the rods. It will be appreciated that materials other than those described above could also be used

While the disclosure has been illustrated and described in detail in the drawings and foregoing description, the same is to be considered as illustrative and not restrictive in character. It should be understood that only the preferred embodiments have been shown and described and that all changes and modifications that come within the scope of the claims are desired to be protected.

## Claims

1. A connector apparatus (30) for linking elongated members, comprising:
a first connector member (32) having a base portion (42) and a post (44), said base portion including first and second channels (54, 56) offset from said post;
a second connector member (34) having a hole (65) and third and fourth channels (66, 68) offset from said hole; and
a locking member (36) connected to said post and adapted to press said second member toward said first member,
said connector members (32, 34) being adapted to be connected so that said post (44) extends through said hole (65) and said connector members (32, 34) are movable with respect to each other, and when connected said connector members define a first location for accommodating a portion of a first elongated member (R1) and a second location for accommodating a portion of a second elongated member (R2), said locations being between said connector members (32, 34) and on respective sides of said post (44);
wherein said first and third channels (54,66) together form a passage (38) at said first location accommodating a portion of said first elongated member (R1), said second and fourth channels (56, 68) together form a passage (40) at said second location for accommodating a portion of said second elongated member (R2) and **characterised in that**
said elongated members (R1, R2) have a diameter larger than the diameter of said passages (38, 40) such that, in use, said elongated members contact edges of the channel (54, 56, 66, 68) ;
and **in that** said post (44) includes at least one longitudinal groove (59), and said second member (34) includes at least one boss (72) extending into said hole (65) and sized and configured to slide along said post groove (59).

2. The apparatus of claim 1, wherein said post (44) is threaded, and said locking member (36) is an internally-threaded nut.

3. The apparatus of claim 1, wherein said post (44) includes two of said longitudinal grooves (59) diametrically opposed to each other, and said second member (34) includes two of said bosses (72) extending into said hole (65) and diametrically opposed to each other, said bosses (72) being sized and configured to slide along respective ones of said post grooves (59).

4. The apparatus of claim 1, wherein said base portion (42) of said first member (32) has a central portion and two side portions (48, 50), and said second member (34) has a central portion and two side portion (63, 64) and wherein at least one of said base portion (42) of said first member (32) and said second member (34) have substantially a diamond shape in which the side portions are narrower than the central portion.

5. The apparatus of claim 1, wherein said base portion (42) of said first member (32) has a central portion and two side portions (48, 50), said post (44) being located in said central portion, and wherein a first of said side portions includes said first substantially linear channel (54) at said first location that substantially faces said second member (34) and is sized and configured to accommodate at least part of an elongated member (R1), and a second of said side portions includes said second substantially linear channel (56) at said second location that substantially faces said second member (34) and is sized and configured to accommodate at least part of an elongated member (R2).

6. The apparatus of claim 5, wherein said channels (54, 56, 66, 68) are substantially parallel.

7. The apparatus of claim 5, wherein said post (44) is substantially between said channels (54, 56, 66, 68).

8. he apparatus of claim 5, wherein second member (34) has a central portion and two side portion (63, 64), said hole (65) being located in said central portion of said second member (34), and wherein said second member (34) includes said third substantially linear channel (66) at said first location that substantially faces said first channel (54) and said fourth substantially linear channel (68) at said second location that substantially faces said second channel (68).

9. The apparatus of claim 8, wherein a first elongated member (R1) is at least partially disposed within one of said passages (38, 40) and a second elongated member (R2) is at least partially disposed within the other of said passages (38, 40).

10. A spinal orthopedic implant apparatus comprising:
the connector apparatus of any of the preceding claims; and first and second spinal rods (R1, R2), being said elongated members,
wherein said locking member (36) is fitted around at least a portion of said post (44) and engages said second connector member (34), whereby said first and second connector members (32, 34) and said first and second spinal rods (R1, R2) are substantially immovable with respect to each other.

11. The apparatus of claim 10, wherein said post (44) and said second connector member (34) include parts of a keying mechanism (59, 72) that substantially limits rotation of said second connector member (34) with respect to said post (44), but does not limit translation of said second connector member (34) with respect to said post (44).

12. The apparatus of claim 11, wherein said keying mechanism includes at least one substantially linear groove (59) in said post (44) and at least one boss (72) on said second connector member (34) positioned at least partially within said at least one groove (59).

13. The apparatus of claim 10, wherein said post (44) is externally threaded and said locking member (36) is a nut.

14. The apparatus of claim 13, wherein said nut includes an undersurface (76) that contacts at least one of said spinal rods (R1, R2).

## Patentansprüche

1. Verbindungsvorrichtung (30) zur Verbindung elongierter Elemente miteinander, wobei die Vorrichtung folgendes umfasst:
ein erstes Verbindungselement (32) mit einem Basisteilstück (42) und einem Pfosten (44), wobei das genannte Basisteilstück erste und zweite Kanäle (54, 56) aufweist, die von dem genannten Pfosten versetzt sind;
ein zweites Verbindungselement (34) mit einer Öffnung (65) und dritten und vierten Kanälen (66, 68), die von der genannten Öffnung versetzt sind; und
ein Verriegelungselement (36), das mit dem genannten Pfosten verbunden ist und das genannte zweite Element in Richtung des genannten ersten Elements drückenkann;
wobei die genannten Verbindungselemente (32, 34) so verbundenwerden können, dass sich der genannte Pfosten (44) durch die genannte Öffnung (65) erstreckt, und wobei die genannten Verbindungselemente (32, 34) im Verhältnis zueinander beweglich sind, und wenn die genannten Verbindungselemente miteinander verbunden sind, definieren sie eine erste Position zur Aufnahme eines Teilstückseines ersten elongierten Elements (R1) sowie eine zweite Position zur Aufnahme eines Teilstückseines zweiten elongierten Elements (R2), wobei sich die genannten Stellen zwischenden genannten Verbindungselementen (32, 34) und auf entsprechenden Seiten des genannten Pfostens (44) befinden;
wobei die genannten ersten und dritten Kanäle (54, 66) gemeinsam einen Durchgang (38) an der genannten ersten Stelle zur Aufnahme eines Teilstücksdes genannten ersten elongierten Elements (R1) bilden, wobei die genannten zweiten und vierten Kanäle (56, 68) gemeinsam einen Durchgang (40) an der genannten zweiten Stelle zur Aufnahme eine' Teilstücksdes genannten zweiten elongierten Elements (R2) bilden, und **dadurch gekennzeichnet, dass**:
die genannten elongierten Elemente (R1, R2) einen Durchmesser aufweisen, der größer ist als der Durchmesser der genannten Durchgänge (38, 40), so dass die genannten elongierten Elemente im Einsatz Kanten der Kanäle (54, 56, 66, 68) berühren;
und wobei der genannte Pfosten (44) mindestens eine longitudinale Rille (59) aufweist, und wobei das genannte zweite Element (34) mindestens einen Vorsprung (72) aufweist, der sich in die genannte Öffnung (65) erstreckt und so bemessen und konfiguriert ist, dass er entlang der genannten Rille (59) des Pfostens gleitet.

2. Vorrichtung nach Anspruch 1, wobei der genannte Pfosten (44) ein Gewinde aufweist, und wobei es sich bei dem genannten Verriegelungselement (36) um eine Mutter mit Innengewinde handelt.

3. Vorrichtung nach Anspruch 1, wobei der genannte Pfosten (44) zwei der genannten longitudinalen Rillen (59) aufweist, die genau entgegengesetzt zueinander angeordnet sind, und wobei das genannte zweite Element (34) zwei der genannten Vorsprünge (72) aufweist, die sich in die genannte Öffnung (65) erstrecken und genau entgegengesetzt zueinander angeordnet sind, wobei die genannten Vorsprünge (72) so bemessen und konfiguriert sind, dass sie entlang entsprechender der genannten Rillen (59) des Pfostens gleiten.

4. Vorrichtung nach Anspruch 1, wobei das genannte Basisteilstück (42) des genannten ersten Elements (32) ein zentrales Teilstückund zwei Seitenteilstücke (48, 50) aufweist, und wobei das genannte zweite Element (34) ein zentrales Teilstückund zwei Seitenteilstücke (63, 64) aufweist, und wobei zumindest eines der genannten Basisteilstücke (42) des genannten ersten Elements (32) und des genannten zweiten Elements (34) im Wesentlichen eine Rautenform aufweist, wobei die Seitenteilstücke schmaler sind als das zentrale Teilstück.

5. Vorrichtung nach Anspruch 1, wobei das genannte Basisteilstück (42) des genannten ersten Elements (32) ein zentrales Teilstückund zwei Seitenteilstücke (48, 50) aufweist, wobei der genannte Pfosten (44) in dem genannten zentralen Teilstück angeordnet ist, und wobei ein erstes der genannten Seitenteilstücke den genannten ersten im Wesentlichen linearen Kanal (54) an der genannten ersten Stelle aufweist, der im Wesentlichen zu dem genannten zweiten Element (34) ausgerichtet und so bemessen und konfiguriert ist, dass sie zumindest einen Teil eines elongierten Elements (R1) aufnimmt, und wobei ein zweites der genannten Seitenteilstücke den genannten zweiten im Wesentlichen linearen Kanal (56) an der genannten zweiten Stelle aufweist, die im Wesentlichen zu dem genannten zweiten Element (34) ausgerichtet und so bemessen und konfiguriert ist, dass sie zumindest einen Teil eines elongierten Elements (R2) aufnimmt.

6. Vorrichtung nach Anspruch 5, wobei die genannten Kanäle (54, 56, 66, 68) im Wesentlichen parallel sind.

7. Vorrichtung nach Anspruch 5, wobei sich der genannte Pfosten (44) im Wesentlichen zwischenden genannten Kanälen (54, 56, 66, 68) befindet.

8. Vorrichtung nach Anspruch 5, wobei das zweite Element (34) ein zentrales Teilstück und zwei Seitenteilstücke (63, 64) aufweist, wobei die genannte Öffnung (65) in dem genannten zentralen Teilstückdes genannten zweiten Elements (34) angeordnet ist, und wobei das genannte zweite Element (34) den genannten dritten im Wesentlichen linearen Kanal (66) an der genannten ersten Stelle aufweist, welche im Wesentlichen zu dem genannten ersten Kanal (54) ausgerichtet ist, und wobei sich der genannte vierte im Wesentlichen lineare Kanal (68) an der genannten zweiten Stelle befindet, die im Wesentlichen zu dem genannten zweiten Kanal (68) ausgerichtet ist.

9. Vorrichtung nach Anspruch 8, wobei sich ein erstes elongiertes Element (R1) zumindest teilweise in einem der genannten Durchgänge (38, 40) befindet, und wobei sich ein zweites elongiertes Element (R2) zumindest teilweise in dem anderen der genannten Durchgänge (38, 40) angeordnet befindet.

10. Implantatvorrichtung für die Wirbelsäulenorthopädie, wobei die Vorrichtung folgendes umfasst:
die Verbindungsvorrichtung nach einem der vorstehenden Ansprüche; und erste und zweite Wirbelsäulenstangen (R1, R2), bei denen es sich um die genannten elongierten Elemente handelt;
wobei das genannte Verriegelungselement (36) zumindest um ein Teilstückdes genannten Pfostens (44) angepasst ist und mit der genannten zweiten Verbindungsvorrichtung (34) eingreift, wodurch die genannten ersten und zweiten Verbindungselemente (32, 34) und die genannten ersten und zweiten Wirbelsäulenstangen (R1, R2) im Verhältnis zueinander im Wesentlichen unbeweglich sind.

11. Vorrichtung nach Anspruch 10, wobei der genannte Pfosten (44) und das genannte zweite Verbindungselement (34) Teile eines Keilverbindungsmechanismus (59, 72) sind, der im Wesentlichen die Rotation des genannten zweiten Verbindungselements (34) im Verhältnis zu dem genannten Pfosten (44) beschränkt, jedoch nicht die Translation des genannten zweiten Verbindungselements (34) im Verhältnis zu dem genannten Pfosten (44) beschränkt.

12. Vorrichtung nach Anspruch 11, wobei der genannte Verkeilungsmechanismus mindestens eine im Wesentlichen lineare Rille(59) in dem genannten Pfosten (44) aufweist sowie mindestens einen Vorsprung (72) an dem genannten zweiten Verbindungselement (34), positioniert zumindest teilweise innerhalb der genannten mindestens einen Rille (59).

13. Vorrichtung nach Anspruch 10, wobei der genannte Pfosten (44) ein Außengewinde aufweist, und wobei es sich bei dem genannten Verriegelungselement (36) um eine Mutter handelt.

14. Vorrichtung nach Anspruch 13, wobei die genannte Mutter eine Unterseite (76) aufweist, die mindestens eine der genannten Wirbelsäulenstangen (R1, R2) berührt.

## Revendications

1. Appareil connecteur (30) pour relier des éléments allongés, comprenant :
un premier élément connecteur (32) ayant une partie de base (42) et un montant (44), ladite partie de base comprenant des premier et deuxième canaux (54, 56) décalés par rapport audit montant ;
un second élément connecteur (34) ayant un trou (65) et des troisième et quatrième canaux (66, 68) décalés par rapport audit trou ; et
un élément de verrouillage (36) relié audit montant et adapté pour presser ledit second élément vers ledit premier élément,
lesdits éléments connecteurs (32, 34) étant adaptés pour être reliés de sorte que ledit montant (44) s'étend à travers ledit trou (65) et lesdits éléments connecteurs (32, 34) sont mobiles l'un par rapport à l'autre, et lorsqu'ils sont reliés, lesdits éléments connecteurs définissent un premier emplacement pour accueillir une partie d'un premier élément allongé (R1) et un second emplacement pour accueillir une partie d'un second élément allongé (R2), lesdits emplacements étant entre lesdits éléments connecteurs (32, 34) et sur les côtés respectifs dudit montant (44) ;
dans lequel lesdits premier et troisième canaux (54, 66) forment ensemble un passage (38) au niveau dudit premier emplacement pour accueillir une partie dudit premier élément allongé (R1), lesdits deuxième et quatrième canaux (56, 68) forment ensemble un passage (40) au niveau dudit second emplacement pour accueillir une partie dudit second élément allongé (R2) et **caractérisé en ce que**
lesdits éléments allongés (R1, R2) ont un diamètre plus grand que le diamètre desdits passages (38, 40) de sorte que, en cours d'utilisation, lesdits éléments allongés entrent en contact avec les bords desdits canaux (54, 56, 66, 68) ;
et **en ce que** ledit montant (44) comprend au moins une rainure longitudinale (59), et ledit second élément (34) comprend au moins un bossage (72) s'étendant dans ledit trou (65) et dimensionné et configuré pour glisser le long de ladite rainure de montant (59).

2. Appareil selon la revendication1, dans lequel ledit montant (44) est fileté, et ledit élément de verrouillage (36) est un écrou à intérieur fileté.

3. Appareil selon la revendication 1, dans lequel ledit montant (44) comprend deux desdites rainures longitudinales (59) diamétralement opposées l'une à l'autre, et ledit second élément (34) comprend deux desdits bossages (72) s'étendant dans ledit trou (65) et diamétralement opposés l'un à l'autre, lesdits bossages (72) étant dimensionnés et configurés pour glisser le long de celles respectives desdites rainures de montant (59).

4. Appareil selon la revendication 1, dans lequel ladite partie de base (42) dudit premier élément (32) a une partie centrale et deux parties latérales (48, 50), et ledit second élément (34) a une partie centrale et deux parties latérales (63, 64), et dans lequel au moins l'une de ladite partie de base (42) dudit premier élément (32) et dudit second élément (34) a sensiblement une forme en losange dans lequel les parties latérales sont plus étroites que la partie centrale.

5. Appareil selon la revendication 1, dans lequel ladite partie de base (42) dudit premier élément (32) a une partie centrale et deux parties latérales (48, 50), ledit montant (44) étant situé dans ladite partie centrale, et dans lequel une première desdites parties latérales comprend ledit premier canal sensiblement linéaire (54) au niveau dudit premier emplacement qui fait sensiblement face audit second élément (34) et est dimensionné et configuré pour accueillir au moins une partie d'un élément allongé (R1), et une seconde desdites parties latérales comprend ledit deuxième canal sensiblement linéaire (56) au niveau dudit second emplacement qui fait sensiblement face audit second élément (34) et est dimensionné et configuré pour accueillir au moins une partie d'un élément allongé (R2).

6. Appareil selon la revendication 5, dans lequel lesdits canaux (54, 56, 66, 68) sont sensiblement parallèles.

7. Appareil selon la revendication 5, dans lequel ledit montant (44) est sensiblement entre lesdits canaux (54, 56, 66, 68).

8. Appareil selon la revendication 5, dans lequel le second élément (34) a une partie centrale et deux parties latérales (63, 64), ledit trou (65) étant situé dans ladite partie centrale dudit second élément (34), et dans lequel ledit second élément (34) comprend ledit troisième canal sensiblement linéaire (66) au niveau dudit premier emplacement qui fait sensiblement face audit premier canal (54) et ledit quatrième canal sensiblement linéaire (68) au niveau dudit second emplacement qui fait sensiblement face audit deuxième canal (68).

9. Appareil selon la revendication 8, dans lequel un premier élément allongé (R1) est au moins partiellement disposé à l'intérieur de l'un desdits passages (38, 40) et un second élément allongé (R2) est au moins partiellement disposé à l'intérieur de l'autre desdits passages (38, 40).

10. Appareil d'implant rachidien orthopédique comprenant :
l'appareil connecteur selon l'une quelconque des précédentes revendications ; et des première et seconde tiges rachidiennes (R1, R2), qui sont lesdits éléments allongés,
dans lequel ledit élément de verrouillage (36) est monté autour d'au moins une partie dudit montant (44) et vient en prise avec ledit second élément connecteur (34), moyennant quoi lesdits premier et second éléments connecteurs (32, 34) et lesdites première et seconde tiges rachidiennes (R1, R2) sont sensiblement immobiles les uns par rapport aux autres.

11. Appareil selon la revendication 10, dans lequel ledit montant (44) et ledit second élément connecteur (34) comprennent des parties d'un mécanisme de manipulation (59, 72) qui limite sensiblement la rotation dudit second élément connecteur (34) par rapport audit montant (44) mais ne limite pas la translationdudit second élément connecteur (34) par rapport audit montant (44).

12. Appareil selon la revendication 11, dans lequel ledit mécanisme de manipulation comprend au moins une rainure sensiblement linéaire (39) dans ledit montant (44) et au moins un bossage (72) sur ledit second élément connecteur (34) positionné au moins partiellement à l'intérieur de ladite au moins une rainure (59).

13. Appareil selon la revendication 10, dans lequel ledit montant (44) est à extérieur fileté, et ledit élément de verrouillage (36) est un écrou.

14. Appareil selon la revendication 13, dans lequel ledit écrou comprend une face inférieure (76) qui entre en contact avec l'au moins une desdites tiges rachidiennes (R1, R2).
